Europäisches Patentamt

European Patent Office

Office européen des brevets

(11) Publication number: **0 036 259**
**B1**

# EUROPEAN PATENT SPECIFICATION

(45) Date of publication of patent specification: **14.06.89**

(21) Application number: **81300858.8**

(22) Date of filing: **02.03.81**

(51) Int. Cl.⁴: **C 12 N 15/00,** C 12 P 21/00, C 12 N 9/86, C 12 N 1/20 // C12R1/125, C12R1/19

(54) **Method, vectors and organisms for transported cloned heterologous gene products in bacillus subtilis and E.coli.**

(30) Priority: **10.03.80 US 128537**
**31.12.80 US 221800**

(43) Date of publication of application:
**23.09.81 Bulletin 81/38**

(45) Publication of the grant of the patent:
**14.06.89 Bulletin 89/24**

(84) Designated Contracting States:
**BE CH DE FR GB IT LI LU NL SE**

(73) Proprietor: **CETUS CORPORATION**
**1400 Fifty-Third Street**
**Emeryville California 94608 (US)**

(72) Inventor: **Chang, Shing**
**118 Golden Rod Drive**
**Hercules California (US)**

(74) Representative: **Allard, Susan Joyce et al**
**BOULT, WADE & TENNANT 27 Furnival Street**
**London EC4A 1PQ (GB)**

(56) References cited:
EP-A-0 006 694
EP-A-0 012 078
EP-A-0 012 494
EP-A-0 021 468
EP-A-0 026 598
EP-A-0 034 470
EP-A-0 038 182

PROCEEDINGS OF THE NATIONAL ACADEMY OF SCIENCE, vol. 75, no. 3, March 1978 (US). T.J. GRYCZAN et al.: "Construction and Properties of Chimeric Plasmids in Bacillus Subtilis", pages 1428-1432

(56) References cited:
NATURE, vol. 279, May 3, 1979, Macmillan Journals Ltd. C.J. BURRELL et al.: "Expression in Escherichia Coli of Hepatitis B Virus DNA Sequences cloned in Plasmid pBR322", pages 43-47

COMPTES RENDUS DE L'ACADEMIE DES SCIENCES DE PARIS, vol. 288, January 15, 1979, serie D. C. MARCHAL et al.: "Biologie Moléculaire - Un systême de vecteurs destiné à permettre l'excrétion d'une protéine déterminée par un gène cloné", pages 275-277

Courier Press, Leamington Spa, England.

(56) References cited:

JOURNAL OF BACTERIOLOGY, vol. 145, no. 1, Januar 1981. O. GRAY et al.: "Molecular Cloning and Expression of Bacillus Licheniformis B-Lactamase Gene in Escherichia Coli and Bacillus Subtilis", pages 422-428

MICROBIOLOGY ABSTRACTS, Section A, vol. 16, no. 2, 1981, ref. no. 1786 A 16, page 110, OXFORD (GB). P.S. LOVETT: "Molecular cloning in Bacillus Subtilis using Plasmid Vectors"

PROCEEDINGS OF THE NATIONAL ACADEMY OF SCIENCE, vol. 77, no. 6, June 1980 (US). K. TALMADGE et al.: "Eukaryotic Signal Sequence Transports Insulin Antigen in Escherichia Coli", pages 3369-3373

CHEMICAL ABSTRACTS, vol. 92, no. 17, April 28, 1980, page 276, abstract no. 140382p, COLUMBUS, Ohio (US) & J. Bacteriol. 1980, 141(1), 246-53. T. GRYCZAN et al.: "Characterization of Chimeric Plasmid Cloning vehicles in Bacillus Subtilis"

CHEMICAL ABSTRACTS, vol. 95, no. 5, August 1981, page 396, abstract no. 38930c, COLUMBUS, Ohio (US) & Nucleic Acids Proteins, Proc. Symp. 1979 (pub. 1980) 529-39. W. GOEBEL et al.: "Molecular Cloning in Bacillus Subtilis"

BIOCHEMICAL AND BIOPHYSICAL RESEARCH COMMUNICATIONS, vol. 91, no. 4, December 28, 1979, Academic Press, Inc. Y. YONEDA et al.: "Cloning of a Foreign Gene Coding for alpha-amylase in Bacilillus Subtilis", pages 1556-1564

CHEMICAL ABSTRACTS, vol. 93, no. 25, December 22, 1980, page 465, abstract no. 234975u, COLUMBUS, Ohio (US) & Plasmic 1979, 2(2), 279-89. A.G. SHIVAKUMAR et al.: "Studies on the Synthesis of Plasmid-Coded Proteins and their Control in Bacillus Subtilis Minicells"

CHEMICAL ABSTRACTS, vol. 93, no. 5, August 4, 1980, page 480, abstract no. 41300m, COLUMBUS, Ohio (US) & Methods Enzymol. 1979, 68 (Recomb. DNA), 342-57. P.S. LOVETT et al.: "Bacillus Subtilis as a Host for Molecular Cloning"

## Description

This invention relates to molecular biology and, more particularly, to the so-called art of recombinant DNA. Specifically, the invention relates to a method and vectors for the production of transported cloned heterologous gene products in *Bacillus subtilis* or *E. coli,* thereby facilitating the recovery of the gene product.

The invention describes novel genetically engineered plasmids. Examples of these organisms have been deposited with the American type Culture Collection, Rockville, Maryland, 20852. They have been assigned ATCC numbers: 31,776—31,778. ATCC number 31,776 has been assigned to plasmid pOG1196; number 31,777 has been assigned to plasmid pOG2165; and number 31,778 has been assigned to plasmid pOG2110.

As is well known, the particular sequence of amino acids in a given protein is determined in accordance with the code carried in the gene for that protein. In the process of translation by which proteins are formed from DNA, via messenger RNA, groups of three nucleotides in the DNA, called codons, each place one of twenty possible amino acids at a corresponding position in the protein chain.

With the advent of recombinant DNA techniques, genetic changes may be made deliberately by the introduction of a predetermined nulceotide sequence, either synthesized or isolated from one strain or species, into the genetic makeup of another strain or species. The known nucleotide sequence may be selected to cause the strain or species into which it is introduced to produce, as part of the translation process, the protein encoded by the known nucleotide sequence. When the modified strain or species proceeds with the normal replication process, it also then duplicates the inserted sequence.

Recombinant DNA techniques involve isolating a suitable piece of DNA chain (a cloning vector) and breaking or severing the two strands of DNA of the cloning vector at the desired location where the foreign DNA is to be inserted. To do this, particular types of proteins, called restriction enzymes, are typically used. Restriction enzymes will break the DNA at particular nucleotide sequences, although with some restriction enzymes the break may not necessarily occur at the same point on the two intertwined DNA strands. In such a case, if two different types of DNA are severed in a similar manner, the open ends will be complementary and will, under suitable conditions, stick together with the complementary ends lying side by side. They may then be linked together enzymatically (with ligase). This makes it possible to recombine two DNA segments from any source into a single DNA molecule.

Once the DNA vector has been isolated and the foreign piece inserted therein, the recombinant DNA is then placed into a suitable host organism. In order for the host organism to replicate the inserted DNA, it is necessary that the recombinant DNA be inserted into the host in such a way as to become part of its genetic system.

For example, in the bacterium *Escherichia coli,* two convenient types of cloning vectors have been utilized. *E. coli* Bacteria, in addition to the main DNA chain or chromosome, frequently have one or more independently replicating circular loops of DNA known as plasmids. Also, a certain type of virus known as a lambda bacteriophage (phage) is also capable of infecting *E. coli* and becoming part of its genetic system. Recombinant DNA techniques have included the use of a variety of plasmids or phages as cloning vectors. This involves the isolation of plasmids or phages from the bacteria, the breaking open of the isolated DNA by restriction enzymes, the insertion of a foreign or heterologous piece of DNA into the plasmid or phage, the restoration of the circular form of the plasmid or the phage structure, and the return of the plasmid or phage to the *E. coli* cell. Once in the host, the heterologous DNA is not only replicated from generation to generation, but also will produce the protein for which it codes if the proper reading frame and promoters exist.

Once heterologous DNA has been successfully recombined into a host microorganism, and the microorganism has produced the cloned gene product, the desired product must be recovered. To do this it has, up to the present invention, been necessary to destroy the cells producing the desired product in order to harvest the product itself. Also, because cells naturally contain a great many different proteins, the isolation process for the desired product may be difficult or complex. Finally, the desired product may be detrimental to the host cell, particularly if it is produced at a high level. In some cases, this may result in destruction of the cells, and in other cases it may result in the cells activating a defensive mechanism to degrade the desired product.

Most recombinant DNA work to date has been carried out with *E. coli. E. coli* is a member of the gram negative class of bacteria which contain two layers of membranes enclosing a periplasmic space. Many of the products produced in *E. coli* are secreted into this periplasmic space, if secreted at all. Few products are secreted outside the living cells into the growth media.

On the other hand, *Bacillus subtilis* is a member of the gram positive class of bacteria which contain only a single layer of bacterial membrane. Thus *B. subtilis* can produce large amounts of protein which are secreted directly into the growth media. Although the general approach to gene cloning in *E. coli* is applicable to *B. subtilis,* attempts to produce a useful product of heterologous gene cloned into *B. subtilis* and secreted into the growth media have thus far been unsuccessful. *B. subtilis* is somewhat preferable to *E. coli* because of a greater efficiency for plasmid mediated transformation and because it is non-pathogenic.

According to the present invention there is provided the use of a DNA sequence homologous to B. licheniformis beta-lactamase operator, promoter, and ribosomal binding site sequence as a control sequence in constructing vectors operable in E. coli or B. subtilis.

The invention also provides an expression vector for use in B. subtilis or E. coli, comprising a vector which is capable of autonomous replication in B. subtilis or E. coli, respectively, and having a DNA sequence therein encoding a heterologous protein, said sequence being located and oriented in said vector such as to be under the control of the B. licheniformis beta-lactamase operator, promoter, and ribosomal binding site sequence, expression of said protein being under the control of a transport mechanism by which said protein is secreted by B. subtilis or secreted into the periplasmic space by E. coli respectively.

The invention includes new B. subtilis and E. coli microorganisms (and their production) containing such vectors and the use of these microorganisms in the production of protein non-indigenous thereto.

The invention also provides a vector for secretion and suitable for use in E. coli or B. subtilis comprising a vector capable of autonomous replication in E. coli or B. subtilis, said latter vector comprising an operable B. licheniformis beta-lactamase operator, promoter, and ribosomal binding site sequence, a functional transport signal sequence and a site in reading frame with said signal sequence and available for insertion of a DNA sequence to be expressed under the control of said operable B. licheniformis beta-lactamase operator, promoter, and ribosomal binding site sequence and said transport signal sequence.

Yet a further aspect provides the use of a DNA seque encoding a prokaryotic beta-lactamase signal sequence as a transport signal sequence in constructing vectors operable in E. coli or B. subtilis.

The invention further includes a vector for secretion and suitable for use in E. coli or B. subtilis comprising a vector capable of autonomous replication in E. coli or B. subtilis, said latter vector comprising an operable operator, promoter and ribosomal binding site sequence, a DNA sequence for a transport signal peptide functional to secrete beta-lactamase from B. subtilis and a site in reading frame with said signal sequence and available for insertion of a DNA sequence to be expressed under the control of said operable operator, promoter, and ribosomal binding site sequence and said transport signal sequence.

The invention will now be further described and illustrated in the following description, taken in connection with the accompanying drawings wherein:

Figure 1 is a schematic drawing providing a partial structural map of a DNA fragment which contains the gene for beta-lactamase obtained from Bacillus licheniformis;

Figure 2 is a schematic drawing indicating the correspondence between a number of the nucleotide sequences in the fragment of Figure 1 and protein sequence present in the protein for which the fragment codes, and also indicating the corresponding restriction enzyme recognition sites;

Figure 3 is a picture indicating the typical appearance of plates upon which Bacillus subtilis strains harboring the recombinant plasmids are grown, as the plates appear after PVA assay for beta-lactamase activity;

Figure 4 is a schematic illustration of the construction of E. coli plasmid pOG2110, B. subtilis plasmid pOG1196, and the bifunctional plasmid pOG2165;

Figure 5 is a picture indicating the typical appearance of plates upon which various bacterial strains harboring the recombinant plasmids are grown, as the plates appear after PVA assay for beta-lactamase activity; and

Figure 6 is a diagram of the nucleotides comprising a portion of the B. licheniformis Pen P gene.

Very generally, and in accordance with one form of the invention, a predetermined protein which is non-indigenous to B. subtilis is produced through expression by B. subtilis. Growth media and conditions are provided for growing a strain of B. subtilis in which a plasmid has been introduced. The plasmid is capable of being replicated in the strain and has a gene therein for the predetermined protein. The gene is located and oriented in the plasmid such as to be under the control of an operator, promoter and ribosomal binding site sequence. The protein is also under the control of a transport mechanism by which the protein is secreted by the host strain. Upon secretion, the protein is recovered from the growth media.

When, according to the invention, the predetermined protein is expressed in a gram negative bacteria, such as E. coli, the protein is again transported across the bacterial membrane by the transport peptide. However, here the protein is "secreted" into the periplasmic space instead of the growth media since the gram negative bacterium has a cell wall in addition to its normal bacterial membrane. The predetermined protein can be recovered from the periplasmic space.

The method of the invention necessitates the use of a cloning vector organism containing a sequence of nucleotides capable of initiating the transcription and translation process. These nucleotides, which provide an operator, promoter, and ribosomal binding site sequence, may be naturally present in the vector, may be inserted therein as a separate segment of DNA using recombinant DNA techniques, or may be part of the heterologous DNA containing the gene of interest. The heterologous DNA, which will contain at least the structural gene for a desired protein product, is placed in the cloning vector so as to be transcribed and translated under control of the operator, promoter, and ribosomal binding site sequence. For correct translation of the inserted heterologous DNA, the nucleotides in the inserted DNA must be in the correct reading frame. In addition, it may be desirable or even necessary that the cloning vector include a sufficient number of nucleotides indigenous to the host cell to ensure readthrough translation from the operator, promoter, and ribosomal binding site sequence into and through the inserted heterologous DNA in the correct reading frame.

In accordance with the invention, the cloning vector utilized includes a sequence of nucleotides which comprise codons for a functional transport signal peptide sequence. Transport signal peptide sequences are typically short leader sequences of amino acids on newly made proteins. Although the mechanism by which the transport signal peptide sequence operates is not entirely understood, it is believed that the transport protein is excreted by the cell and withdraws that protein appended to it from the cytoplasm as the protein is made. Once the transport function has been performed by the transport signal peptide sequence, the transport sequence may be removed by natural processes.

In accordance with the invention, the heterologous DNA can be inserted into the cloning vector at a location which allows the protein for which it encodes to be transported in accordance with the transport signal peptide sequence coded by the signal codons. Thus, the cloned gene product can be conveniently transported to a desired destination from which the gene product may be harvested. This has several advantages. Because the destination is outside the cell in the case of *B. subtilis*, and in the periplasmic space in the case of *E. coli*, the host cells need not be destroyed to harvest the gene products, thus allowing in the former for the continuous or uninterrupted production of the gene production. Also, since cells contain a great many proteins, the ability to export the cloned gene product makes the isolation and purification of the product a much simpler task. Finally, since cloned gene products, especially if produced at a high level, may be detrimental to the host cells, the ability to harvest the cloned gene products from outside of the cell membranes (if not, in the case of *E. coli*, the cell wall) often means that the products will not harm the cells, nor will the cells possibly produce a defensive enzyme which will degrade the gene product.

The precise location at which the heterologous DNA must be inserted in the cloning vector will, of course, depend upon how the transport signal peptide sequence functions. In some cases, the transport signal peptide sequence will immediately precede the heterologous DNA, either as part of the gene itself or having already been present in the plasmid. The signal sequence itself can constitute the necessary sequence of nucleotides to provide for readthrough translation of the heterologous DNA. On the other hand, there may be some cases in which the transport signal peptide sequence must be located elsewhere then immediately preceding the heterologous DNA. In such cases, it may be necessary to produce the desired peptide sequence with some additional amino-acids at the beginning (coded by the extraneous codons) in order to provide the necesary readthrough functions.

The following examples illustrate specific instances in which the invention may be employed, but are not intended to limit the scope of the invention:

Example 1

Construction of a plasmid for producing a transported predetermined protein non--indigenous to a host organism

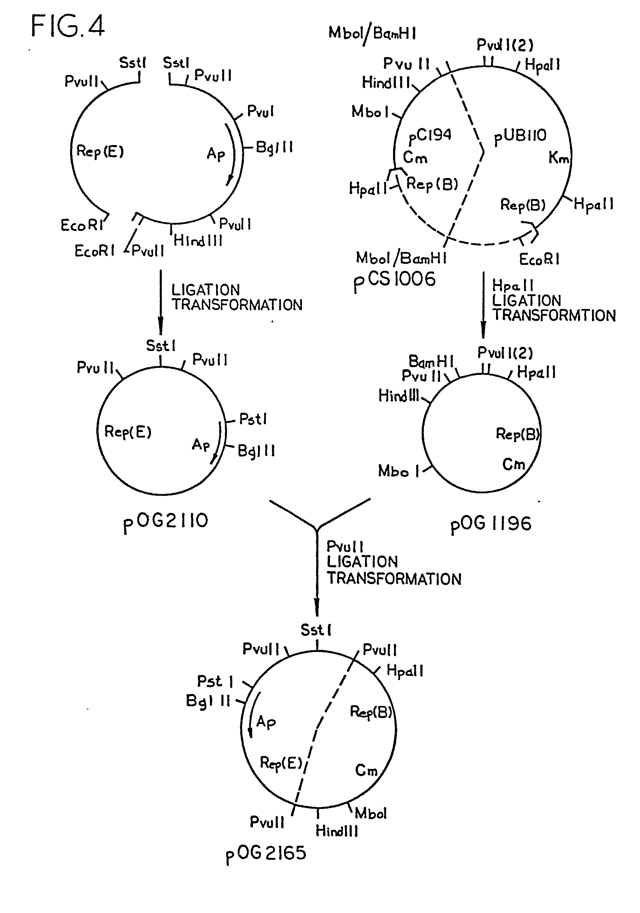

In order to provide a plasmid for producing a predetermined protein non-indigenous to a host organism, a plasmid vector containing the *B. licheniformis* beta-lactamase gene was made and then replicated in both *B. subtilis* and *E. coli*. The plasmid was constructed by purifying a 3.5 kb (kilobase) EcoRI-SstI fragment containing the beta-lactamase gene and then ligating it with a 2.1 kb. EcoRI-SstI fragment containing the replication function of the *E. coli* plasmid pOP1Δ6 (described by Gelfand, et al., *Proc. The Nat. Acad. Sci. USA*, (1978) *75,* 5869—5873). Following transformation into competent *E. coli* CS412 cells (an $r_k^- m_k^+ Pro^-$ derivative of C600) and a growth period adequate for expression (90 minutes), Ap-resistant transformants were obtained. Plasmids from three clones were prepared; one designated as pOG2110 was further characterized. Figure 4 details the location of the expected and observed restriction sites in pOG2110.

To allow for replication of pOG2110 in *B. subtilis,* a bifunctional replicon was constructed using pOG2110 and the *B. subtilis* plasmid pOG1196. The construction of pOG1196 is summarized in Figure 4.

Initially a chimeric plasmid (pCS832) containing the entire sequences of plasmids pC194 (Cm^R) and pUB110 (Km^R) was made by ligating the two MboI fragments of pC194 with the BamHI digested pUB110. The resulting plasmid carried both the Cm gene from PC194 and the km (Nm) gene from pUB110. It has a size of 7.5 kb. A spontaneous deletion mutant (plasmid pCS1006) was obtained from one of the sub-clones. It had lost the HpaII site originated in pC194 and known to be located in the pC194 replication region (Chang and Cohen, *Molec. Gen. Genet.,* (1979) *168,* 111—115). It still retained the replication function of pUB110 and the two resistance markers. By recircularizing the largest HpaII fragment (3.6 kb) of pCS1006, plasmid pOG1196 was obtained. This plasmid confers only Cm-resistance and possesses the replication function derived from plasmid pUB110. The map of pOG1196 is shown in Figure 4.

*E. coli* plasmid pOG2110 and *B. subtilis* plasmid pOG1196 contained two and three PvuII sites respectively. Equal amounts of PvuII digested pOG2110 and pOG1196 plasmid DNA were ligated and used to transform *E. coli* strain CS412. Cm-resistant clones were selected; all were also Ap-resistant. The composite plasmid pOG2165 isolated from one of the Cm-resistant Ap-resistant transformants was studied further. A map of this 7.5 kb plasmid is shown in Figure 4. Plasmid pOG2165 replicates in both *E. coli* and *B. subtilis* and confers upon either host both Cm- and Ap-resistance.

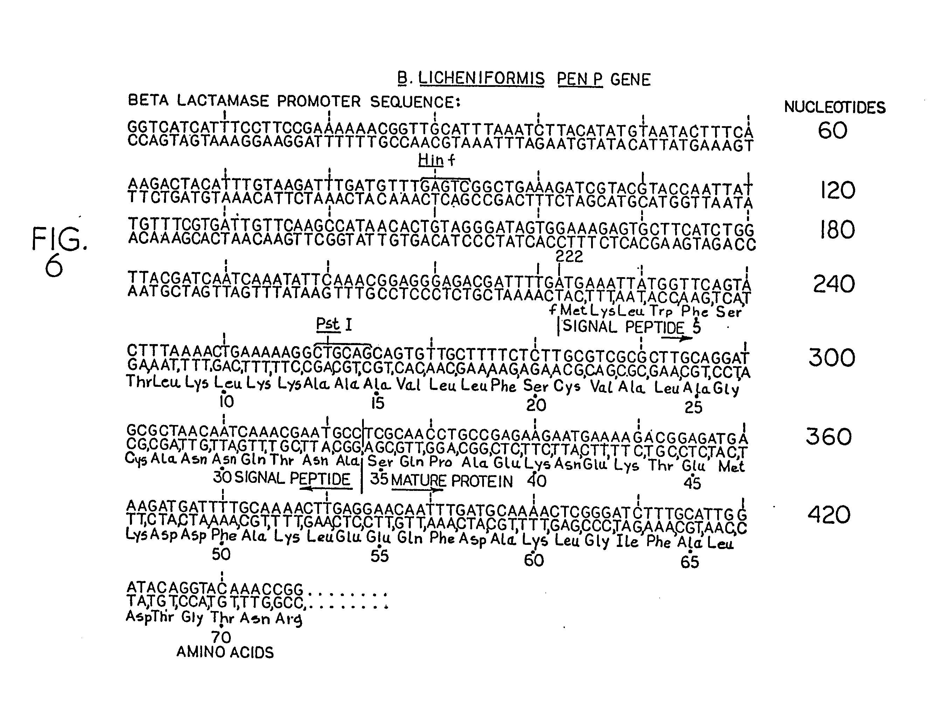

*B. subtilis* and *E. coli* cells harboring plasmid pOG2165 are resistant to ampicillin as a result of the production of the *B. licheniformis* beta-lactamase enzyme. This can be demonstrated by means of the PVA plate assay developed by Sherratt and Collins, *J. Gen. Microbiol.*, (1973), *76* 217—230. The positive results obtained from such an assay are illustrated in Figure 5.

When pOG2165 is propagated in *B. subtilis* BD224, both the membrane-bound and the secreted form of the heterologous beta-lactamase are synthesized. The amount produced by this strain is variable and depends upon the growth conditions used. pOG2165 can also be propagated in *B. subtilis* strain QB127 (Kunst, et al., *Bio. Chemie.* (1974) *56,* 1481—1490). QB127 is a bacterial strain with the sacU^h mutation which causes overproduction of several exoenzymes such as levansucrase, alpha-amylase and extra-cellular proteases. Levels of beta-lactamase detected in cultures of QB127 (pOG2165) are similar to those levels detected in BD224 (pOG2165) cultures under the same conditions.

The bi-functional plasmid pOG2165 itself possesses unique sites for restriction enzymes *Sst*I, *Hind*III, *Pst*I and *Bg*III. Insertion of DNA into the *Bg*III and the *Pst*I sites leads to inactivation of the *B. licheniformis* beta-lactamase gene and provides an easily recognizable phenotype for identifying clones carrying inserts.

When the exact reading frame of the DNA sequence to be inserted is known, it is possible to create a fused protein containing the leader sequence and the first 71 amino acid residues of the beta-lactamase exoenzyme by cloning into the *Bg*III site. Fused protein made in this way is secreted by the *Bacillus* cells due to the presence of the leader sequence at the amino terminus. These features make pOG2165 a useful vector for the cloning and efficient expression of heterologous genes, and the subsequent secretion of the gene product in *B. subtilis* and *E. coli.*.

On the other hand, when the exact reading frame of the DNA sequence to be inserted is known, and insertion is made at the *Pst*I site, a fused protein is made which will accumulate in the host organism. Since the *Pst*I recognition site is located in the initial portion of the nucleotide sequence coding for the signal peptide, only a portion of this sequence is transcribed before the heterologous gene sequence is encountered. Even though the fused protein is expressed, this portion of the signal peptide is insufficient to confer the normal signal peptide's secretion function. As a result, products of genes inserted at the *Pst*I site accumulate in the host organism.

A portion of the nucleotide sequence comprising the *B. licheniformis* Pen P gene is diagrammed in Figure 6. The beta-lactamase promoter region is located between nucleotides 1 and 221 one; its precise location is unknown. The nucleotides which code for the amino acids comprising the signal peptide being with nucleotide 222 and end with nucleotide 323. As a result, the signal peptide is composed of thirty-four amino acids. The *Pst*I recognition site is located between nucleotide positions 259 and 264, or at amino acids thirteen through fifteen on the signal peptide chain. Insertion of heterologous DNA into this *Pst*I site will lead to the formation of a fused protein composed of the heterologous DNA product and the first fourteen amino acids of the signal peptide chain. Such a fused protein will be expressed but not transported across the bacterial membrane. Successful secretion requires fusion with either the total signal peptide, or fusion with at least the first twenty-six amino acid residues in the signal peptide chain.

Example 2

*B. licheniformis* produces a large amount of beta-lactamase in the secreted form (exo-enzyme). The secretion of this protein is believed to be result of the interaction between the bacterial membrane and the amino acid leader sequence which facilitates the transport of the protein across the single bacterial membrane barrier. The beta-lactamase gene was cloned and inserted into plasmids which were capable of being replicated in *B. subtilis.* The plasmids were then transformed into the *B. subtilis* hosts resulting in the secretion of beta-lactamase. This constitutes the first expression of a heterologous gene in *B. subtilis* and the transport of the gene products into the culture or growth media from the *B. subtilis* cells.

To clone the beta-lactamase gene from the *B. licheniformis* strain, total chromosomal DNA from *B. licheniformis* strain 749/C was isolated and digested with *Eco*RI restriction endonuclease. Chromosomal DNA isolated from *B. licheniformis* 749/C was prepared according to Marmur (*J. of Molec. Biol.* (1961) *3,* 208—18). *E. coli* plasmid pSC101 is isolated from cells using the cleared lysate procedure of Kupersztoch and Helinski (*Biochem. Biophys. Res. Commun.* (1973) *54,* 1451—59). 3 µg of chromosomal DNA and 2 µg of pSC101 DNA were digested with endonuclease *Eco*RI and ligated with T4 DNA ligase as described (Hershfield, et al. *Proc. Natl. Acad. Sci., USA,* (1974) *71,* 3455—59), and transformed into competent cells of *E. coli* strain CS412 (an $r_k^-m_k^+Pro^-$ derivative of C600) using the protocol of Cohen, et al. (*Proc. Natl. Acad. Sci., USA,* (1972) *69,* 2110—14).

Transformants resistant to ampicillin at 10 µg/ml were selected and one of the transformants carrying recombinant plasmid designated pTB2 was characterized further. Plasmid pTB2 carries a 4.2 kb (kilobase pair) *Eco*RI fragment on the pSC101 vector. This plasmid confers to the host tetracycline (the marker on pSC101) and ampicillin resistances, indicating that the beta-lactamase gene product is made as a functional enzyme which degrades ampicillin in the media.

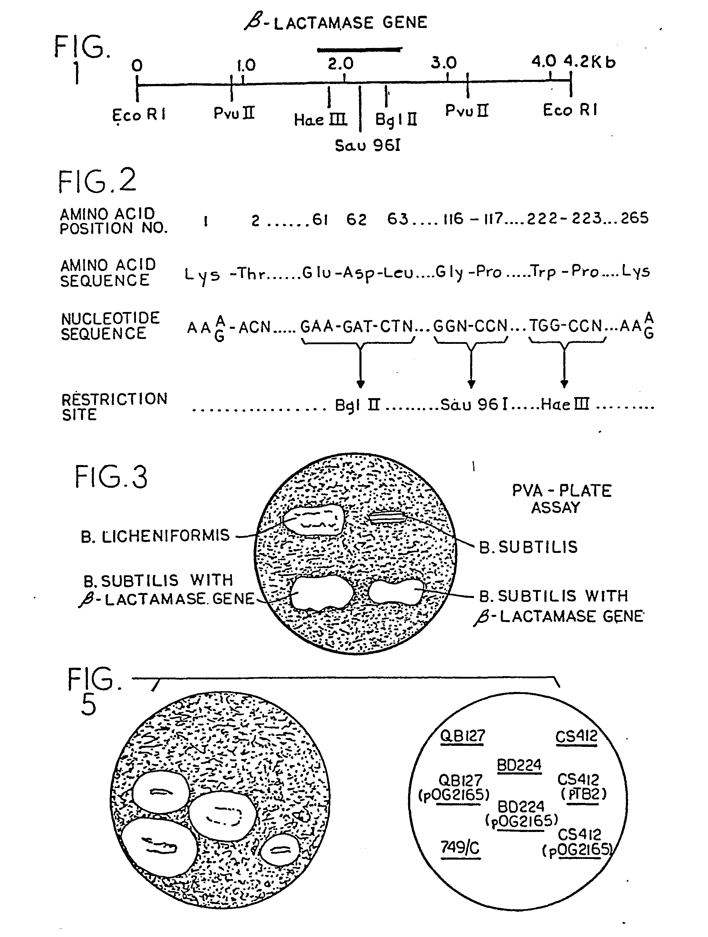

The beta-lactamase gene is located on the 4.2 kilobase pair *Eco*RI fragment mentioned above. Subsequent analysis of this fragment using various restriction enzymes and gene cloning permits deduction of the structure of the gene for this enzyme as partially mapped and shown in Figure 1. The primary sequence of the beta-lactamase from *B. licheniformis* strain 749/C has been previously determined (R. J. Meadway, Ph.D., Thesis, University of Edinburgh, 1969). From the known amino acid sequence, the Gly-Pro (position 116—117) sequence corresponds to the nucleotide sequence GGN-CCN, which in turn is the recognition sequence for endonuclease *Sau*96I (GGNCC). Similarly, the Trp-Pro (position 222—223) sequence is coded by nucleotide codons TGG-CCN, within which the center tetranucleotide sequence GGCC is recognised and cleaved by endonuclease *Hae*III (see Roberts, in *DNA Insertion Elements, Plasmids, and Episomes,* 1977, ed. Bukhari, Shapiro and Adhya, Cold Spring Harbor Lab., p 757).

The 4.2 kb cloned fragment was analyzed by a number of endonuclease, as listed in Figure 2, using the conditions specified by the supplier (New England Biolabs, Inc., Beverly, Ma 01915, 1978 catalog). The digested DNA was analyzed on agarose gels as described by Sharp, et a. (*Biochemistry* (1973) *12,* 3055—63), and on acrylamide gels (Maxam and Gilbert, *Proc. Natl. Acad. Sci., USA,* (1973) *73,* 3942—46). The mapping data is summarized in Figure 2. The *Sau*961 site and the *Hae*III site were located in the 2.3 kb *Pvu*II fragment which contains the complete beta-lactamase gene sequence. These two sites are separated by 320 nucleotides which is consistent with the protein sequence data (106 amino acids apart).

After identification of the nucleotide sequence which contains the beta-lactamase gene, the gene was cloned into *B. subtilis* using various *Bacillus* plasmids and using a hybrid *B. subtilis-E. coli* plasmid. Plasmids included pUB110 and pC221 derived plasmids. The *B. subtilis* strains harboring the recombinant plasmids become resistant to ampicillin as well as give a positive beta-lactamase reaction on PVA plates (see Figure 3). Furthermore, beta-lactamase activity was detected in cultures after bacterial cells were removed. This activity clearly indicates the successful expression of the heterologous gene in *B. subtilis,* as well as the transport of the protein through the bacterial membrane into the culture or growth media.

The *Eco*RI fragment containing the beta-lactamase gene was cloned onto *B. subtilis* plasmid vectors pUB110 (Gryczan and Dubnau, *Proc. Natl. Acad. Sci., USA.,* (1978) *75,* 1428—1432) and pC221 (Ehrlich, *Proc. Natl. AAcad. Sci., USA,* (1977) *74* 1680—82) at the respective *Eco*Ri sites using the procedure described above in connection with *E. coli.* Similarly, the 2.3 kb *Pvu*II fragment containing the beta-lactamase gene has also been cloned onto pUB110 at the *Pvu*II site and the *Tac*I site.

Litigated DNA preparations were used to transform *B. subtilis* strain BD224 (*rec*E4, *trp*C2, *thr*5) by the method of Chang and Cohen, *Molec. Gen.*

*Genet.,* (1979) *168,* 111—115. Transformants resistant to ampicillin on regeneration plates were selected and tested. The production of beta-lactamase is detected by two methods; one is a sensitive plate assay developed by Sherratt and Collins, *J. Gen. Microbiol.,* (1973) 76 217—230, the other is the iodometric assay described by Ross and O'Callaghan, (*Meth. in Enzymology,* (1975), *43,* 69—85. *B. subtilis* clones resistant to ampicillin gave positive results for both tests. In addition, beta-lactamase activity was also detected in the culture after the cells were removed, indicating that the beta-lactamase is not only made, but also exported in *B. subtilis.*

The cloning of heterologous genes into *B. subtilis* and the functional expression of these genes as intracellular proteins have been shown previously by Keggins, et al., *Proc. Natl. Acad. Sci., USA.,* (1978) *75,* 1423—1427. In this study, however, the cloned genes are genes coding for enzymes which are normally present intracellularly in wild type *B. subtilis.* The successful expression of genes which are non-indigenous to *B. subtilis,* and the successful secretion of the products of these genes was not demonstrated.

The work on the beta-lactamase gene presented in this example is the first demonstration that a new function, namely, beta-lactamase production, can be introduced into *B. subtilis* using gene cloning techniques and employing a bacterial beta-lactamase gene control sequence. In addition this Example constitutes the first clear demonstration that a foreign gene product can be made to pass through the *B. subtilis* membrane barrier and be secreted as an exo-protein. The beta-lactamase, which is a commercially useful product, is produced by a strain which does not otherwise produce this enzyme.

Example 3

The *B. licheniformis* beta-lactamase gene described in Example 2 was also inserted into plasmids capable of being replicated in *E. coli.* These plasmids were transformed into the *E. coli* hosts using methods identical to those described in Example 2 for *B. subtilis.* The *E. coli* cells harboring the plasmids are resistant to ampicillin as a result of the production of the *B. licheniformis* beta-lactamase enzyme. This is demonstrated by means of the PVA plate assay developed by Sherratt and Collins, *J. Gen. Microbiol.,* (1973), *76* 217—230.

When the *B. licheniformis* beta-lactamase gene carrying plasmids are propagated in *E. coli,* the secreted form of the enzyme is not transported into the culture medium as it is in *B. subtilis.* Since *E. coli* has a cell wall surrounding the limiting bacterial membrane, the heterologous protein product is transported to the periplasmic space separating the cell wall from the bacterial membrane. The beta-lactamase exoenzyme was allowed to accummulate the periplasmic space and was then harvested by appropriate methods.

## Example 4

The beta-lactamase gene of *B. licheniformis* is not the only source of a signal peptide sequence that can function in *B. subtilis* or *E. coli*. As indicated above, many proteins (especially in eucaryotic cells) are transported across membranes. Although the precise amino acid sequence of the "signal region" may, in fact does, vary among different transported proteins, the folded structures of these regions, which can be predicted according to the rules of Chou and Fasman (*Ann. Rev. Biochem.* (1978) *47*, 251—276, are very similar. Thus, the requirements for a transport signal peptide sequence can be satisfied with non-*Bacillus* signal peptides, provided the signal sequence encoding DNA fragment is correctly positioned downstream from the *B. licheniformis* · beta-lactamase promoter, and ribosomal binding site sequence.

One such eucaryotic transport signal sequence that may be used in connection with a desired gene product is the signal or presequence preceding the insulin B chain. (The various insulin chains, A, B and C, are made as a single polypeptide and are assembled and processed in the endoplasmic reticulum on the way to being exported by the cell). However, in the insulin presequence there is no convenient restriction site immediately after the signal peptide sequence which can be used to join it with a cloned gene of heterologous DNA. Nevertheless, the last five nucleotides of the signal sequence in insulin are AGGCT and the first nucleotide of the insulin B chain itself is T. These six nucleotides together (AGGCTT) differ by a single nucleotide from AAGCTT which is the recognition sequence for the restriction enzyme *Hind*lll. The enzyme cuts between the two As. If the heterologous DNA was cloned or separated using the same enzyme, *Hind*lll, or using a half *Hind*lll site bifunctional linker, the sequence will be restored when the heterologous DNA is inserted.

The single nucleotide $G_x$ may be changed to an A to provide the *Hind*lll site by a procedure in which the nucleotide to be changed is exposed, altered, and the sequence reconstructed. This change results in no alteration of the next to last amino acid of the signal sequence.

DNA litigation may be accomplished as described by Hershfield, et al., *Proc. Natl. Acad. Sci., USA,* (1974) *71*, 3455—3495. Reverse transcriptase may be used as a DNA polymerase such as described by Bahl, et al., *Proc. Natl. Acad. Sci., USA,* (1977) *74*, 966—970, Transformation may proceed as described by Cohen, et al., *Proc. Natl. Acad. Sci., USA,* (1973) *70*, 3240—3244.

## Example 5

Although the procedure described in Example 4 above is technically feasible, it is a eukaryotic leader sequence and thus the procedure may be useful only in connection with general research. Typically, a more useful approach from a commercial standpoint is in connection with a prokaryotic (bacterial) host. Although only a few such signal sequences are known in connection with prokaryotic systems, one sequence which is well characterized is the sequence from TEM beta-lactamase. (Sutcliffe, *Proc. Natl. Acad. Sci., USA,* (1976) *75*, 3737—3741). This sequence is ideal for attaching to a cloned gene except that there is no convenient restriction site at the location for presequence processing. The nearest restriction site to the signal sequence is an *Mbo*l site which would result in the attachment of 16 extraneous amino acids to the cloned gene product.

Nevertheless, the terminal portion of the signal sequence, which is TTTGCT, may be altered, by one of the above-described techniques, to TTTGAT. When the latter sequence is read along with the first few following nucleotides of the TEM beta-lactamase gene, a restriction site for *Bcl*l (TGATCA) exists. This results in the altering of the final amino acid from Ala to Asp and in the attaching of one extra amino acid to the gene product, either a Glu or His, depending upon what the first attached nucleotide of the heterologous DNA is.

To accomplish the foregoing alteration in nucleotides, the approach described above may be used wherein a short fragment is synthesized. There are bracketing restriction sites upstream from the signal sequence (*Tha*l) and a restriction site *Mbo*l, as mentioned above, downstream. A *taq* site exists even further downstream. Restriction conditions may be followed for *Tha*l as described by McConnell, et al., *Nucleic Acid Res.* (1978), *5*, 1729—1739; for *Mbo*l by Gelinas et al., *J. Mol. Biol.* (1977) *114*, 169—179; or *Taq*l by Sato, et al., *Proc. Natl. Acad. Sci., USA,* (1977) *74*, 542—546. Note that in connection with *Mbo*l, the DNA is prepared in the host cell GM119 which lacks deoxyadenosine methylase (to avoid methylation of regions and prevent *Mbo*l cutting). In this latter connection see Marinus and Morris *Mutat. Res.* (1975) *28*, 15—26. Alternatively, the restriction endonuclease SAU 3A, an isoschizome of *Mbo*l, may be used with DNA isolated from any host.

It may be seen, therefore, that the invention provides a method and a vector for the controlled accumulation of heterologous cloned gene products. The products are transferred or transported outside the host cell membrane, enabling the harvesting of the product to proceed with minimal restrictions, and avoiding the likelihood of degradation or destruction of either the host cell or the gene product itself.

References
1. Bahl, C. P., Wu. R., Stawinsky, J. and Narang, S. A., *Proc. Nat. Acad. Sci., USA,* (1977) 74:966—970.
2. Chang, A. and Cohen, S., *Molec. Gen. Genet.,* (1979) 168:111—115.
3. Chou, P. Y. and Fasman, G. D., *Ann. Rev. Biochem.,* (1978), 47:251—276.
4. Cohen, S. N. and Chang, A. C. Y., *Proc. Nat. Acad. Sci., USA,* (1972), 69:2110—2114.
5. Cohen, S. N., Chang, A. C. Y., Boyer, H. W.

and Helling, R. B., *Proc. Nat. Acad. Sci., USA,* (1973) (70:3240—3244.

6. Ehrlich, S. D., *Proc. Nat. Acad. Sci., USA,* (1977) 74:1680—1682.

7. Gelinas, R. E. Myers, P. A. and Roberts, R. J., *J. Molec Biol.,* (1977) 114:169—179.

8. Gelfand, D., Shepard, H., O'Farrell, P. and Polisky, B., *Proc. Nat. Acad. Sci., USA,* (1978) 75:5869—5873.

9. Gryczan, T. J. and Dubnau, D., *Proc. Nat. Acad. Sci. USA,* (1978) 75:1428—1432.

10. Hershfield, V., Boyer, H. W., Yanofsky, C., Lovett, M. A. and Halsinki, D. R., *Proc. Nat. Acad. Sci., USA,* (1974) (71:3455—3459.

11. Keggins, K. M., Lovett, P. S. and Duvall, E. J., *Proc. Nat. Acad. Sci., USA,* (1978) 75:1423—1427.

12. Kunst. F., Pascal, M., Lepesant-Kejzlarova, J., Lepesant, J., Billault, A. and Dedonder, R., *Bio. Chemie.,* (1974) 56:1481—1490.

13. Kupersztoch, Y. and Helinski, D., *Biochem. Biophys. Res. Commun.,* (1973) 54:1451—1459.

14. Marinus, M. G. and Morris. N. R., *Mutat. Res.,* (1975) 28:15—26.

15. Marmur, J., *J. Molec. Biol.,* (1961) 3:208—218.

16. Maxam, A. and Gilbert, W., *Proc. Nat. Acad. Sci., USA,* (1973) 73:3942—3946.

17. McConnell, D. J., Searcy, D. G. and Sutcliffe, J. G., *Nucleic Acids Res.,* (1978) 5:1729—1739.

18. Meadway, R. J. Ph.D. Thesis,' University of Edinburgh, (1969).

19. Roberts, in *DNA Insertion Elements, Plasmids and Episomes,* 1977, editors Bukhari, Shapiro and Adhya, Cold Spring Harbor Lab. p. 757.

20. Ross, G. W. and O'Callaghan, C. H., *Meth. Enzymol.,* (1975) (43:69—85.

21. Sato, S., Hutchison, D. A. III and Harris, J. I., *Proc. Nat. Acad. Sci. USA,* (1977), 74:542—546.

22. Sharp, P. A., Sugden, B. and Sambrook, J., *Biochem.,* (1973) 12:3055—3063.

23. Sherratt, D. J. and Collins, J. F., *J. Gen. Microbiol.,* (1973), 76:217—230.

24. Sutcliffe, J. G., *Proc. Nat. Acad. Sci., USA,* (1978), 75:3737—3741.

**Claims**

1. The use of a DNA sequence homologous to *B. licheniformis* beta-lactamase operator, promoter, and ribosomal binding site sequence as a control sequence in constructing vectors operable in *E. coli* or *B. subtilis.*

2. A vector for secretion and suitable for use in *E. coli* or *B. subtilis* comprising a vector capable of autonomous replication in *E. coli* or *B. subtilis,* said latter vector comprising an operable *B. licheniformis* beta-lactamase operator, promoter, and ribosomal binding site sequence, a functional transport signal sequence and a site in reading frame with said signal sequence and available for insertion of a DNA sequence to be expressed under the control of said operable *B. licheniformis* beta-lactamase operator, promoter, and ribosomal binding site sequence and said transport signal sequence.

3. A vector according to claim 2 wherein said transport sequence is provided for by a portion of a prokaryotic beta-lactamase gene or a portion of a eukaryotic insulin signal peptide gene.

4. A vector according to claim 3 wherein said DNA transport sequence encodes an amino acid sequence exhibiting homology to the *B. licheniformis* beta-lactamase transport signal sequence.

5. A vector according to claim 2 wherein said transport sequence is a DNA sequence encoding a transport signal sequence functional to secrete beta-lactamase from *E. coli* and *B. subtilis* or functional to secrete proinsulin from *E. coli* and *B. subtilis.*

6. A vector according to claim 1 comprising a base sequence of a plasmid selected from the group consisting of pC221, pUB110, pC194, pUB112, pT127, pOG2165, pOG2110, pCS1006, pOG1196, and pCS832.

7. An expression vector for use in *B. subtilis* or *E. coli,* comprising a vector which is capable of antonomous replication in *B. subtilis* or *E. coli,* respectively, and having a DNA sequence therein encoding a heterologous protein, said sequence being located and oriented in said vector such as to be under the control of the *B. licheniformis* beta-lactamase operator, promoter, and ribosomal binding site sequence, expression of said protein being under the control of a transport mechanism by which said protein is secreted by *B. subtilis* or secreted into the periplasmic space by *E. coli,* respectively.

8. A vector according to claim 7 comprising the base sequence of a plasmid selected from the group consisting of: pC221, pUB110, pC194, pUB112, pT127, pOG2165, POG2110, pCS1006, pOG1196, and pCS832.

9. A vector according to claim 7 wherein said heterologous protein is beta-lactamase.

10. A vector according to claim 7 or claim 9 wherein said transport mechanism is provided for by a portion of a prokaryotic beta-lactamase gene or by a portion of a eukaryotic insulin signal peptide gene.

11. A vector according to claim 7 wherein said predetermined protein is a mammalian protein or hormone.

12. A vector according to claim 7 which is bifunctional and capable of expression in both *B. subtilis* and *E. coli.*

13. A method for creating a microorganism capable of producing heterologous protein through expression, comprising using a vector as defined in any one of claims 7 to 12 to transform *B. subtilis* or *E. coli,* as appropriate.

14. A microorganism which is *B. subtilis* or *E. coli* transformed by a vector as defined in any one of claims 7 to 12.

15. A microorganism according to claim 14 wherein the vector is a bifunctional plasmid possessing unique sites for restriction enzymes *Sst*I, *Hind*III, *Pst*I and *Bgl*II.

16. A method for producing protein comprising providing growth conditions in a growth medium for a microorganism according to claim 14 or claim 15 and recovering said heterologous protein.

17. A method for producing vectors comprising growing B. subtilis or E. coli containing a vector as defined in any one of claims 2 to 6 or a vector as defined in any one of claims 7 to 12 under conditions whereby replication of said vector occurs.

18. The use of a DNA sequence encoding a prokaryotic beta-lactamase signal sequence as a transport signal sequence in constructing vectors operable in E. coli or B. subtilis.

19. The use according to claim 18 wherein said transport signal sequence is homologous to the B. licheniformis beta-lactamase transport signal sequence.

20. A vector for secretion and suitable for use in E. coli or B. subtilis comprising a vector capable of autonomous replication in E. coli or B. subtilis, said latter vector comprising an operable operator, promoter and ribosomal binding site sequence, a DNA sequence for a transport signal peptide functional to secrete beta-lactamase from B. subtilis and a site in reading frame with said signal sequence and available for insertion of a DNA sequence to be expressed under the control of said operable operator, promoter, and ribosomal binding site sequence and said transport signal sequence.

**Patentansprüche**

1. Verwendung einer zur Sequenz des β-Lactamase-Operators, Promotors und der Ribosomen-Bindungsstelle von B. licheniformis homologen DNA-Sequenz als Steuersequenz zur Konstruktion von Vektoren, die in E. coli oder B. subtilis funktions fähig sind.

2. Vektor für die Sekretion, der zur Verwendung in E. coli oder B. subtilis geeignet ist, umfassend einen zur autonomen Replikation in E. coli oder B. subtilis befähigten Vektor, wobei der letztere Vektor eine funmionsfähige Sequenz des β-Lactamase-Operators, Promotors und der Ribosomen-Bindungsstelle von B. licheniformis, eine funktionelle Transportsignal-Sequenz und eine Spaltstelle im Leseraster mit der Signalsequenz umfaßt, die für die Insertion einer DNA-Sequenz verfügbar ist, die unter der Kontrolle der funktionsfähigen Sequenz des β-Lactamase-Operators, Promotors und der Ribosomen-Bindungsstelle von B. licheniformis und der Transportsignal-Sequenz exprimiert werden soll.

3. Vektor nach Anspruch 2, in dem die Transportsequenz durch einen Bereich eines prokaryotischen β-Lactamasegens oder einen Bereich eines eukaryotischen Insulin-Signalpeptid-Gens bereitgestellt wird.

4. Vektor nach Anspruch 3, in dem die DNA-Transportsequenz eine Aminosäuresequenz codiert, die Homologie zur β-Lactamase-Transportsignal-Sequenz von B. licheniformis aufweist.

5. Vektor nach Anspruch 2, in dem die Transportsequenz eine DNA-Sequenz ist, die für eine Transportsignal-Sequenz codiert, deren Funktion die Sekretion von β-Lactamase aus E. coli und B. subtilis oder deren Funktion die Sekretion von Proinsulin aus E. coli und B. subtilis ist.

6. Vektor nach Anspruch 1, umfassend eine Basensequenz eines Plasmids aus der Gruppe pC221, pUB110, pC194, pUB112, pT127, pOG2165, pOG2110, pCS1006, pOG1196 und pCS832.

7. Expressionsvektor zur Verwendung in B. subtilis oder E. coli, umfassend einen Vektor, der zur autonomen Replikation in B. subtilis bzw. E. coli befähigt ist und der eine DNA-Sequenz aufweist, die für ein heterologes Protein codiert, wobei diese Sequenz in dem Vektor so angeordnet und orientiert ist, daß sie unter der Kontrolle der Sequenz von β-Lactamase-Operator, Promotor und Ribosomen-Bindungsstelle von B. licheniformis steht, und wobei die Expression des Proteins unter der Kontrolle eines Transportmechanismus steht, durch den das Protein durch B. subtilis sezerniert bzw. durch E. coli in den periplasmatischen Raum sezerniert wird.

8. Vektor nach Anspruch 7, umfassend die Basensequenz eines Plasmids aus der Gruppe pC221, pUB110, pC194, pUB112, pT127, pOG2165, pOG2110, pCS1006, pOG1196 und pCS832.

9. Vektor nach Anspruch 7, in dem das heterologe Proten β-Lactamase ist.

10. Vektor nach Anspruch 7 oder Anspruch 9, in dem der Transportmechanismus durch einen Bereich eines prokaryotischen β-Lactamasegens oder durch einen Bereich eines eukaryotischen Insulin-Signalpeptid-Gens bereitgestellt wird.

11. Vektor nach Anspruch 7, in dem das bestimmte Protein ein Säugerprotein oder -hormon ist.

12. Vektor nach Anspruch 7, welcher bifunktionell und zur Expression sowohl in B. subtilis als auch E. coli in der Lage ist.

13. Verfahren zur Erzeugung eines zur Herstellung eines heterologen Proteins durch Expression befähigten Mikroorganismus, umfassend die Verwendung eines Vektors nach einem der Ansprüche 7 bis 12 zur Transformation von B subtilis oder E. coli, je nach Zweckmäßigkeit.

14. Mikroorganismus, nämlich B. subtilis oder E. coli, transformiert mit einem Vektor nach einem der Ansprüche 7 bis 12.

15. Mikroorganismus nach Anspruch 14, in dem der Vektor ein bifunktionelles Plasmid ist, das einzige Schnittstellen für die Restriktionsenzyme SStI, HindIII, PstI und BgIII aufweist.

16. Verfahren zur Erzeugung eines Proteins, bei dem in einem Wachstumsmedium für einen Mikroorganismus nach Anspruch 14 oder Anspruch 15 Wachstumsbedingungen geschaffen werden und das heterologe Protein gewonnen wird.

17. Verfahren zur Herstellung von Vektoren, bei dem B. subtilis oder E. coli, die einen Vektor nach einem der Ansprüche 2 bis 6 oder einen Vektor nach einem der Ansprüche 7 bis 12 enthalten, unter Bedingungen gezüchtet werden, bei denen eine Replikation des Vektors eintritt.

18. Verwendung einer für eine prokaryotische β-Lactamase-Signalsequenz codierenden DNA-Sequenz als Transportsignal-Sequenz bei der Konstruktion von Vektoren, die in *E. coli* oder *B. subtilis* funktionsfähig sind.

19. Verwendung nach Anspruch 18, wobei die Transportsignal-Sequenz zu der β-Lactamase-Transportsignal-Sequenz von *B. licheniformis* homolog ist.

20. Vektor für die Sekretion, der zur Verwendung in *E. coli* oder *B. subtilis* geeignet ist und einen zur autonomen Replikation in *E. coli* oder *B. subtilis* befähigten Vektor umfaßt, wobei der letztere Vektor eine funktionsfähige Operator-, Promotor- und Ribosomen-Bindungsstellen-Sequenz, eine DNA-Sequenz für ein Transport-Signalpeptid, das die Sekretion von β-Lactamase aus *B. subtilis* bewirkt, und eine Stelle im Leseraster mit der Signalsequenz umfaßt, die für die Insertion einer DNA-Sequenz verfügbar ist, die unter der Kontrolle der funktionsfähigen Operator-, Promotor- und Ribosomen-Bindungsstellen-Sequenz und der Transportsignal-Sequenz exprimiert werden soll.

## Revendications

1. Utilisation d'une séquence d'ADN homologue de la séquence d'opérateur, promoteur et site de liaison ribosomique de la β-lactamase de *B. licheniformis,* en tant que séquence régulatrice dans la construction de vecteurs utilisables dans *E. coli* ou *B. subtilis.*

2. Vecteur pour la secrétion et apte à être utilisé dans *E. coli* ou *B. subtilis,* comprenant un vecteur capable de réplication autonome dans *E. coli* ou *B. subtilis,* ledit vecteur comprenant une séquence utilisable d'opérateur, promoteur et site de liaison ribosomique de la bêta-lactamase de *B. licheniformis,* une séquence signal de transport fonctionnelle et un site en cadre de lecture avec ladite séquence signal et disponible pour l'insertion d'une séquence d'ADN à exprimer sous la régulation de ladite séquence utilisable d'opérateur, promoteur et site de liaison ribosomique de la bêta-lactamase de *B. licheniformis* et de ladite séquence signal de transport.

3. Vecteur selon la revendication 2, dans lequel ladite séquence de transport est fournie par une portion d'un gène de bêta-lactamase de procaryote ou par une portion d'un gène de peptide signal de l'insuline d'encaryote.

4. Vecteur selon la revendication 3, dans lequel ladite séquence d'ADN de transport code pour une séquence d'aminoacides manifestant une homologie avec la séquence signal de transport de la bêta-lactamase de *B. licheniformis.*

5. Vecteur selon la revendication 2, dans lequel ladite séquence de transport est une séquence d'ADN codant pour une séquence signal de transport fonctionnelle pour la secrétion de bêta-lactamase par *E. coli* et *B. subtilis,* ou fonctionnelle pour la secrétion de proinsuline par *E. coli* et *B. subtilis.*

6. Vecteur selon la revendication 1, comprenant une séquence de bases d'un plasmide choisi parmi pC221, pUB110, pC194, pUB112, pT117, pOG2165, pOG2110, pCS1006, pOG1196 et pCS832.

7. Vecteur d'expression à utiliser dans *B. subtilis* ou *E. coli,* comprenant un vecteur qui est capable de réplication autonome dans *B. subtilis* ou *E. coli,* respectivement, et contenant une séquence d'ADN codant pour une protéine hétérologue, ladite séquence étant située et orientée dans ledit vecteur de manière à être sous la régulation de la séquence d'opérateur, promoteur et site de liaison ribosomique de la bêta-lactamase de *B. licheniformis,* l'expresion de ladite protéine étant sous la régulation d'un mécanisme de transport par lequel ladite protéine est secrétée par *B. subtilis* ou secrétée dans l'espace périplasmique par *E. coli,* respectivement.

8. Vecteur selon la revendication 7, comprenant la séquence de bases d'un plasmide choisi parmi pC221, pUB110, pC194, pUB112, pT127, pOG2165, pOG2110, pCS1006, pOG1196 et pCS832.

9. Vecteur selon la revendication 7, dans lequel ladite protéine hétérologue est la bêta-lactamase.

10. Vecteur selon la revendication 7 ou la revendication 9, dans lequel ledit mécanisme de transport est fourni par une portion d'un gène de bêta-lactamase de procaryote ou par une portion d'un gène de peptide signal d'insuline d'eucaryote.

11. Vecteur selon la revendication 7, dans lequel ladite protéine préétablie est une protéine ou une hormone de mammifère.

12. Vecteur selon la revendication 7, qui est bifonctionnel et capable d'expression à la fois dans *B. subtilis* et dans *E. coli.*

13. Procédé pour la création d'un micro-organisme capable de produire une protéine hétérologue par expression, comprenant l'utilisation d'un vecteur tel que défini dans l'une quelconque des revendications 7 à 12, pour la transformation de *B. subtilis* ou *E. coli,* selon ce qu'il convient.

14. Micro-organisme, lequel est *B. subtilis* ou *E. coli,* transformé par un vecteur selon l'une quelconque des revendications 7 à 12.

15. Micro-organisme selon la revendication 14, dans lequelle le vecteur est un plasmide bifonctionnel possédant des sites uniques pour les enzymes de restriction *Sst*I, *Hind*III, *Pst*I et *Bg*III.

16. Procédé pour la production de protéine, comprenant la fourniture de conditions de croissance dans un milieu de croissance pour un micro-organisme selon la revendication 14 ou la revendication 15, et la récupération de ladite protéine hétérologue.

17. Procédé pour la production de vecteurs, comprenant la croissance de *B. subtilis* ou *E. coli* contenant un vecteur tel que défini dans l'une quelconque des revendications 2 à 6 ou un vecteur tel que défini dans l'une quelconque des revendications 7 à 12, dans des conditions dans lesquelles a lieu la réplication dudit vecteur.

18. Utilisation, d'une séquence d'ADN codant pour une séquence signal de bêta-lactamase de procaryote, en tant que séquence signal de transport dans la construction de vecteurs utilisables dans *E. coli* ou *B. subtilis.*

19. Utilisation selon la revendication 18, dans laquelle ladite séquence signal de transport est homologue de la séquence signal de transport de la bêta-lactamase de *B. licheniformis.*

20. Vecteur pour la secrétion et apte à être utilisé dans *E. coli* ou *B. subtilis,* comprenant un vecteur capable de réplication autonome dans *E. coli* ou *B. subtilis,* ce dernier vecteur comprenant une séquence utilisable d'opérateur, promoteur et site de liaison ribosomique, une séquence d'ADN codant pour un peptide signal de transport fonctionnel pour la secrétion de bêta-lactamase par *B. subtilis* et un site en cadre de lecture avec ladite séquence signal et disponible pour l'insertion d'une séquence d'ADN à exprimer sous la régulation de ladite séquence utilisable d'opérateur, promoteur et site de liaison ribosomique, et de ladite séquence signal de transport.

FIG. 1

β-LACTAMASE GENE

FIG.2

| AMINO ACID POSITION NO. | 1 | 2 ......61 62 63.... 116 – 117 .... 222 – 223 ... 265 |

AMINO ACID SEQUENCE: Lys -Thr......Glu-Asp-Leu....Gly-Pro.....Trp-Pro....Lys

NUCLEOTIDE SEQUENCE: AA$^A_G$-ACN..... GAA-GAT-CTN...GGN-CCN...TGG-CCN...AA$^A_G$

RESTRICTION SITE: ...............  Bgl II .........Sau 96 I.....Hae III .........

FIG.3

PVA - PLATE ASSAY

B. LICHENIFORMIS

B. SUBTILIS

B.SUBTILIS WITH β-LACTAMASE GENE

B. SUBTILIS WITH β-LACTAMASE GENE

FIG. 5

QB127
CS412
BD224
QB127 (pOG2165)
CS412 (PTB2)
BD224 (pOG2165)
749/C
CS412 (pOG2165)

FIG.4

pCS1006

pOG2110

pOG1196

pOG2165

B. LICHENIFORMIS PEN P GENE

BETA LACTAMASE PROMOTER SEQUENCE:

NUCLEOTIDES

```
GGTCATCATTTCCTTCCGAAAAAACGGTTGCATTTAAATCTTACATATGTAATACTTTCA          60
CCAGTAGTAAAGGAAGGATTTTTTGCCAACGTAAATTTAGAATGTATACATTATGAAAGT
```

Hinf

```
AAGACTACATTTGTAAGATTTGATGTTTGAGTCGGCTGAAAGATCGTACGTACCAATTAT        120
TTCTGATGTAAACATTCTAAACTACAAACTCAGCCGACTTTCTAGCATGCATGGTTAATA
```

FIG. 6

```
TGTTTCGTGATTGTTCAAGCCATAACACTGTAGGGATAGTGGAAAGAGTGCTTCATCTGG        180
ACAAAGCACTAACAAGTTCGGTATTGTGACATCCCTATCACCTTTCTCACGAAGTAGACC
```
222

```
TTACGATCAATCAAATATTCAAACGGAGGGAGACGATTTTGATGAAATTATGGTTCAGTA        240
AATGCTAGTTAGTTTATAAGTTTGCCTCCCTCTGCTAAAACTACTTTAATACCAAGTCAT
                                              fMetLysLeu Trp Phe Ser
                                              |SIGNAL PEPTIDE 5
```

Pst I

```
CTTTAAAACTGAAAAAGGCTGCAGCAGTGTTGCTTTTCTCTTGCGTCGCGCTTGCAGGAT       300
GAAATTTTGACTTTTTCCGACGTCGTCACAACGAAAAGAGAACGCAGCGCGAACGTCCTA
ThrLeu Lys Leu Lys LysAla Ala Ala Val Leu LeuPhe Ser Cys Val Ala Leu Ala Gly
            10                15                20              25
```

```
GCGCTAACAATCAAACGAATGCCTCGCAACCTGCCGAGAAGAATGAAAAGACGGAGATGA       360
CGCGATTGTTAGTTTGCTTACGGAGCGTTGGACGGCTCTTCTTACTTTTCTGCCTCTACT
Cys Ala Asn Asn Gln Thr Asn Ala Ser Gln Pro Ala Glu Lys AsnGlu Lys Thr Glu Met
         30 SIGNAL PEPTIDE | 35 MATURE PROTEIN 40                45
```

```
AAGATGATTTTGCAAAACTTGAGGAACAATTTGATGCAAAACTCGGGATCTTTGCATTGG       420
TTCTACTAAAACGTTTTGAACTCCTTGTTAAACTACGTTTTGAGCCCTAGAAACGTAACC
LysAsp Asp Phe Ala Lys LeuGlu Glu Gln Phe Asp Ala Lys Leu Gly Ile Phe Ala Leu
         50                55                60              65
```

```
ATACAGGTACAAAACCGG........
TATGTCCATGTTTTGGCC.........
AspThr Gly Thr Asn Arg
         70
         AMINO ACIDS
```

EP 0 036 259 B1